# EUROPEAN PATENT SPECIFICATION

(11) **EP 3 450 567 B1**
(45) Date of publication and mention of the grant of the patent: **18.12.2019**
(21) Application number: 17189155.9
(22) Date of filing: 04.09.2017
(51) Int. Cl.: C12P 17/04, C12P 7/62, C12N 9/02

(54) **PROCESS FOR THE PREPARATION OF (R)-BETA-ANGELICA LACTONE FROM ALPHA-ANGELICA LACTONE EMPLOYING ENE-REDUCTASES**
VERFAHREN ZUR HERSTELLUNG VON (R)-BETA-ANGELICALACTON AUS ALPHA-ANGELICALACTON UNTER VERWENDUNG VON EN-REDUKTASEN
PROCÉDÉ DE PRODUCTION DE (R)-BÊTA-ANGELICA LACTONE À PARTIR D'ALPHA-ANGELICA LACTONE EN UTILISANT D'ÈNE RÉDUCTASES

(43) Date of publication of application: 06.03.2019
(73) Proprietor: F.I.S.- Fabbrica Italiana Sintetici S.p.A., 36075 Montecchio Maggiore (VI) (IT)
(72) Inventor: Motterle, Riccardo, 36057 Arcugnano Vicenza (IT); Bergantino, Elisabetta, 35010 Cadoneghe Padova (IT); Robescu, Marina Simona, 21020 Mornago Varese (IT); Niero, Mattia, 30035 Mirano Venezia (IT); Fogal, Stefano, 36040 Brendola Vicenza (IT)
(74) Representative: Leganza, Alessandro

(56) References cited:
- WU, Y ET AL.: "Asymmetric Olefin Isomerization of Butenolides via Proton Transfer Catalysis by an Organic Molecule", JOURNAL OF THE AMERICAN CHEMICAL SOCIETY, vol. 133, no. 32, 18 July 2011 (2011-07-18), pages 12458-12461, XP002778664, DOI: 10.1021/ja205674x -& WU, Y. ET AL.: "Supporting Information - Asymmetric olefin isomerization of butenolides via proton transfer catalysis by an organic molecule", , 18 July 2011 (2011-07-18), XP002778665, Retrieved from the Internet: URL:https://pubs.acs.org/doi/suppl/10.1021 /ja205674x/suppl_file/ja205674x_si_002.pdf [retrieved on 2018-02-28]
- TURRINI, N.G. ET AL.: "Sequential Enzymatic Conversion of alpha-Angelica Lactone to gamma-Valerolactone through Hydride-Independent C=C Bond Isomerization", CHEMSUSCHEM, vol. 9, no. 24, 25 November 2016 (2016-11-25), pages 3393-3396, XP002778666, DOI: 10.1002/cssc.201601363
- DATABASE Protein [Online] 26 September 2013 (2013-09-26), SCHOENKNECHT, G. ET AL.: "NADPH2 dehydrogenase-like protein [Galdieria sulphuraria]", XP002778667, retrieved from NCBI Database accession no. XP_005703492
- DATABASE Protein [Online] 2 October 2015 (2015-10-02), ANONYM.: "alkene reductase [Chroococcidiopsis thermalis]", XP002778668, retrieved from NCBI Database accession no. WP_015152687

## Description

### Technical Field

The present invention relates to a new and efficient process for the preparation of chiral β-angelica lactone being (*R*)-β-angelica lactone, by reaction of α-angelica lactone with ene-reductase enzymes.

### Background Art

The chemical compound 5-methylfuran-2(5H)-one, also known as β-angelica lactone, is a small molecule having the following chemical formula:

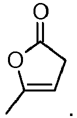

Due to the asymmetry of the carbon atom bonded to the methyl group, said compound shows (*R*) or (*S*) configuration thus having the following formulas: 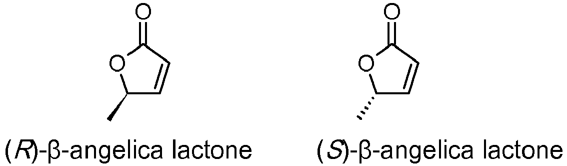 or can exist as mixture thereof in any ratio or can be a racemic compound, i.e. a 50% mixture of optical isomers R and *S,* being represented by the first chemical formula above.

The compound β-angelica lactone is a versatile building block with several applications, for example as precursor of biobased polymers and natural products.

Moreover, as disclosed by Amos B. Smith III et al. in Journal of the American Chemical Society, (1995), 117, pag. 10755-10756, (*R*)-β-angelica lactone can be used as building block for the synthesis of (+)-3-Epifuraquinocin C and (-)-Furaquinocin C, active compounds having respectively the following formulas:

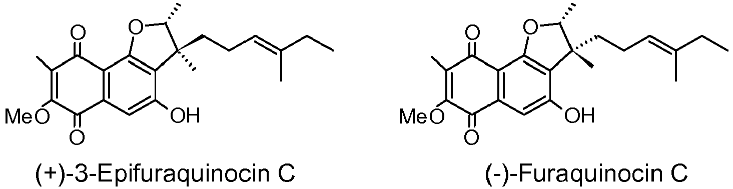

the latter shows wide-ranging biological effects, such as antihypertensive activity, and inhibition of platelet aggregation and coagulation.

Moreover, β-angelica lactone is a precursor of γ-valerolactone of formula: 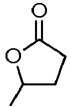 which, as the β-angelica lactone, can have (*R*) or (*S*) configuration, thus having the following chemical formulas: 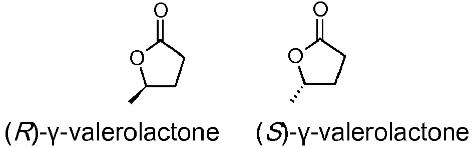 or can exist as mixture thereof or can be a racemic compound. Finally, γ-valerolactone is also abbreviated as GVL (which stands for Gamma-Valerolactone) and therefore, its enantiomers are known and abbreviated as (*R*)-GVL and (*S*)-GVL.

The enantiomers of γ-valerolactone, i.e. (*R*)-GVL or (*S*)-GVL, can be they selves building blocks for the synthesis of natural products and biologically active molecules such as pheromones, polyketides and prostaglandins, as well as pharmaceutical active compounds, e.g. the antihypertensive named WS75624, and the antileukemic Steganacin. Moreover, said chiral γ-valerolactones can be used in the pheromone synthesis, for instance for the synthesis of optically active forms of Sulcatol.

Chiral γ-valerolactone can be obtained by means of several processes and starting from different starting materials, such as, for instance, those disclosed by József M. Tukacs et al. Green Chemistry, (2015) 17, pag. 5189-5195, wherein (*S*)-GVL is produced by ring closure of the precursor (*S*)-4-hydroxyvaleric acid, which was produced by asymmetric reduction of levulinic acid or, alternatively, it can be prepared by enantioselective reduction, catalysed by chiral Rodium complex catalysts, of other substrates.

The bioenzimatic preparation of racemic β-angelica lactone has been disclosed in literature, specifically the article ChemSusChem (2016), 9, pag. 3393-3396. In said article racemic β-angelica lactone is obtained by isomerization from racemic α-angelica lactone, which was further reduced to γ-valerolactone in a cascade from α-angelica lactone, through β-angelica lactone, as reported in the reaction scheme below.

The first part of said process, i.e. the isomerization of α-angelica lactone to β-angelica lactone, is carried out by OYEs in a buffer solution, wherein OYEs are ene-reductases from the Old Yellow Enzyme (OYE), and, in particular, by means of OYE2, which is an ene-reductase capable to isomerize the double bound. The product so obtained, with a conversion of about 45 %, is a racemic product. Moreover, said isomerization of the double bond is carried out also by others OYEs, such as those chosen in the group of: OYE2, OYE3, NCR, YqjM, OPR1, OPR3, EBP1, XenA; e.g. in the Table 2, entry 3, said reaction is carried out by OYE3 with conversion of about 35%. However, all the above mentioned ene-reductases only provide racemic compounds.

The following reaction scheme summarizes what beforehand described of the above-mentioned article: 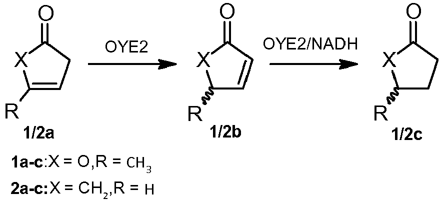 wherein all the compounds are racemic, as stated by the authors at page 3394.

Steven A. King in Journal of Organic Chemistry, pag. 2253-2256, 59, (1994), describes several routes of synthesis of chiral 4-methoxy pentanoic esters, one of them starting from (*R*)-γ-valerolactone, as reported in the following reaction scheme:

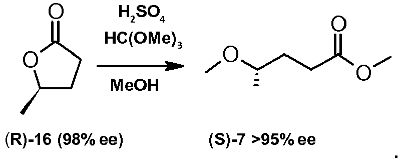

Moreover, Journal of the Am. Chem. Soc., 2011, 133, p. 12458-12461, discloses an isomerization with catalyst that produces a compound (9a) in 90% ee. Further, the related "Supporting Information" describes a process for the preparation of compound 9a with 90% ee.

### Summary of invention

The problem addressed by the present invention is therefore that of providing a new and efficient process for the preparation of chiral β-angelica lactone, in particular for the preparation of (*R*)-β-angelica lactone and derivatives thereof, such as (*R*)-GVL and (*R*)-4-hydroxypentanoic acid.

The process of the present invention starts from racemic α-angelica lactone, involves the isomerization of the double bonds, and, at the same time and surprisingly, induces the chirality thus providing chiral (*R*)-β-angelica lactone.

This problem is solved by a process for the preparation of (*R*)-β-angelica lactone as outlined in the annexed claims, whose definitions are integral part of the present description.

The present invention provides a process for the preparation of (*R*)-β-angelica lactone of formula (I): 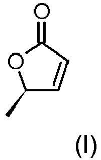 by means of the reaction of α-angelica lactone of formula (II): 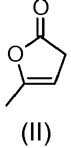 with an ene-reductase having SEQ. ID n. 1 or SEQ. ID n. 2.

Another aspect is a process for the preparation of (*R*)-γ-valerolactone of formula (III): 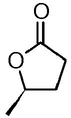 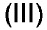 wherein said compound is prepared by further reduction of the (*R*)-β-angelica lactone of formula (I) previously prepared according to the present invention.

Another aspect is a process for the preparation of (*R*)-4-hydroxypentanoic acid of formula (IV) or salts thereof: 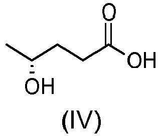 which is produced by hydrolysis the compound of formula (III), obtained as beforehand described.

Furthermore, disclosed are the ene-reductases having SEQ. ID n. 1 or SEQ. ID n. 2.

### Drawings

Fig. 1 shows the amino acid sequence SEQ. ID n. 1, the ene-reductase enzyme from Galdieria sulphuraria, modified according to the present invention.
Fig. 2 shows the amino acid sequence SEQ. ID n. 2, the ene-reductase enzyme from Chroococcidiopsis thermalis, modified according to the present invention.

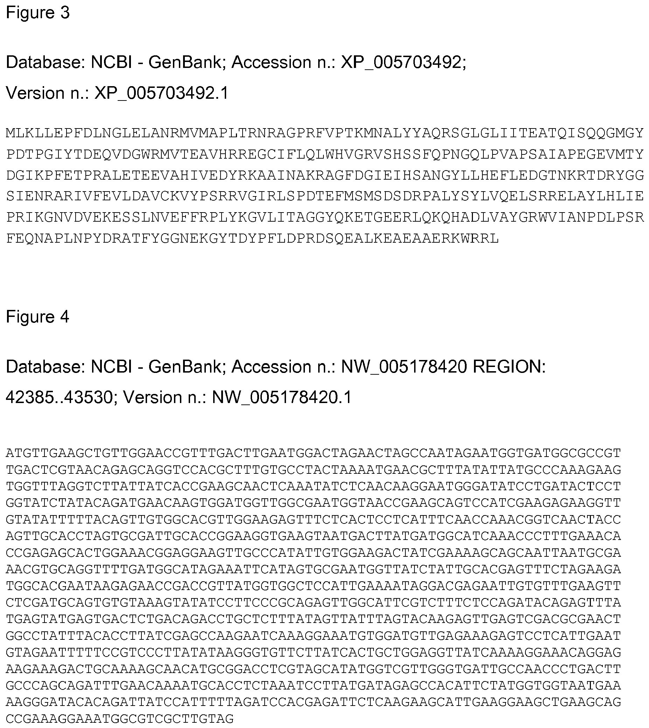

Fig. 3 shows the amino acid sequence SEQ. ID n. 1a of the ene-reductase from the Galdieria sulphuraria.
Fig. 4 shows the nucleotide sequence SEQ. ID n. 1b of the ene-reductase from the Galdieria sulphuraria, that encodes for the amino acid sequence SEQ. ID n. 1a.
Fig. 5 shows the amino acid sequence SEQ. ID n. 2a of the ene-reductase from the Chroococcidiopsis thermalis.
Fig. 6 shows the nucleotide sequence SEQ. ID n. 2b of the ene-reductase from the Chroococcidiopsis thermalis, that encodes for the amino acid sequence SEQ. ID n. 2a.

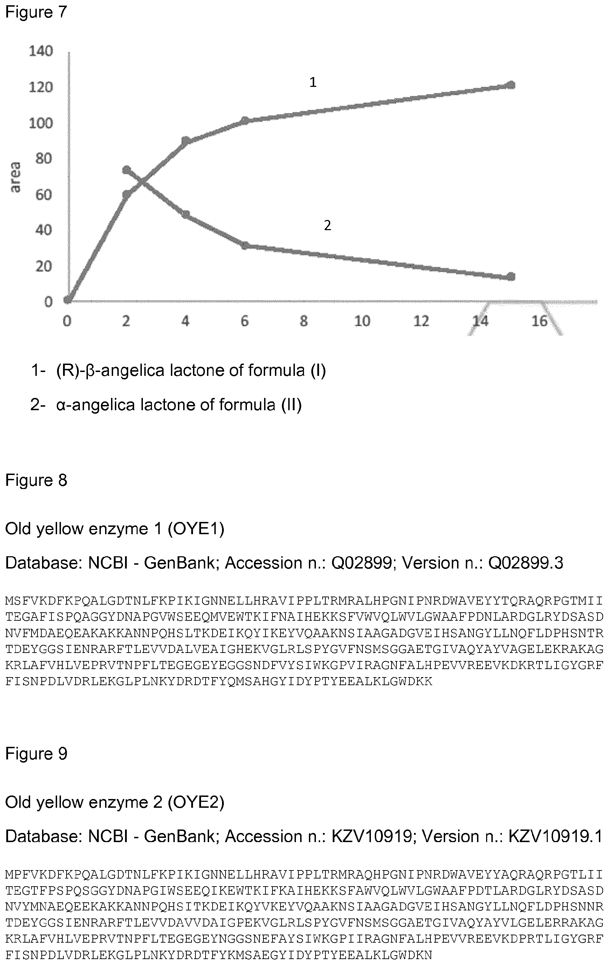

Fig. 7 shows the conversion plot of α-angelica lactone of formula (II) to (R)-β-angelica lactone of formula (I).
Fig. 8 shows the amino acid sequence of the Old Yellow enzyme 1 (OYE1) (also known NADPH dehydrogenase 1) an ene-reductase enzyme produced from *Saccharomyces pastorianus.*
Fig. 9 shows the amino acid sequence of the Old Yellow enzyme 2 (OYE2) an ene-reductase enzyme produced from *Saccharomyces cerevisiae.*

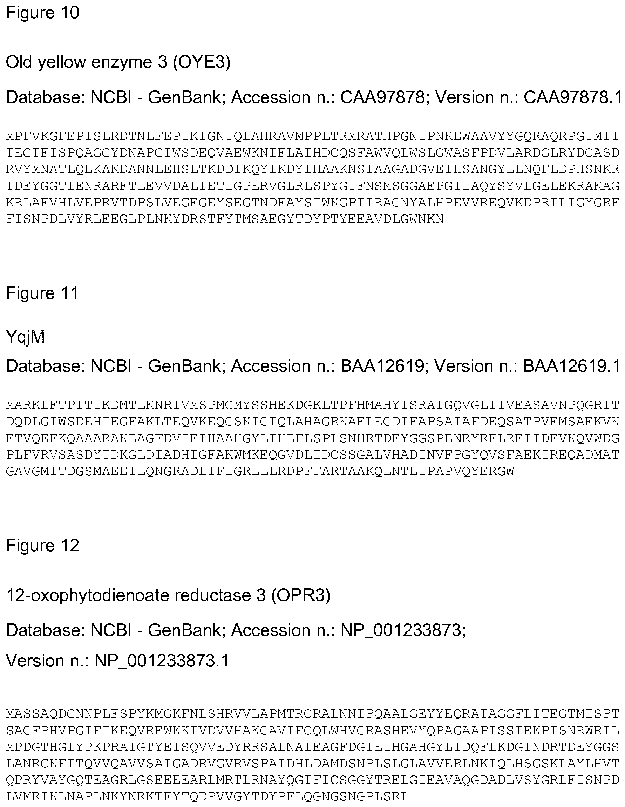

Fig. 10 shows the amino acid sequence of the Old Yellow enzyme 3 (OYE3) an ene-reductase enzyme produced from *Saccharomyces cerevisiae.*
Fig. 11 shows the amino acid sequence of the Old Yellow enzyme homologue (YqjM) an ene-reductase enzyme produced from *Bacillus subtilis.*
Fig. 12 shows the amino acid sequence of the 12-oxophytodienoate reductase 3 (OPR3) an ene-reductase enzyme produced from *Solanum lycopersicum.*
Fig. 13 shows SDS-PAGE analysis and immunoblotting analysis of the ene-reductases having SEQ. ID n. 1 and SEQ. ID n. 2

### Description of embodiments

The stereo-selective reduction of prochiral double bonds for the preparation of optically active molecules is one of most studied and analyzed reactions to prepare chiral products, such as specific enantiomers or diasteroisomers.

The prior art describes many reduction reactions carried out by means of chemical chiral catalysts to prepare chiral compounds such as (*R*)-β-angelica lactone and (*R)*-γ-valerolactone. Nevertheless, when the same reactions are carried out by means of enzymes, as catalysts, they provide only racemic compounds.

Contrarily, the object of the present invention is an efficient process for the preparation of (*R*)-β-angelica lactone of formula (I): 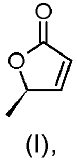 by means of the reaction of α-angelica lactone of formula (II): 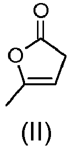 with an ene-reductase having SEQ. ID n. 1 or SEQ. ID n. 2.

It has been indeed surprisingly found that new ene-reductases, having SEQ. ID n. 1 or SEQ. ID n. 2, provide the stereo-selective isomerization of the double bond, thus producing the optically active (*R*)-β-angelica lactone and then, by further reduction, (*R*)-γ-valerolactone.

The following scheme summarizes the invention:

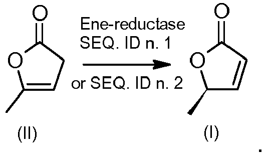

Furthermore, the reaction of the present invention from α-angelica lactone of formula (II) to (*R*)-β-angelica lactone of formula (I) by means of the beforehand mentioned ene-reductases, proceeds with excellent regioselectivity and stereoselectivity.

In particular, with reference to regioselectivity, said reaction occurs without the formation of the other compounds, as chemical impurities, and provides only a clean conversion from the compound of formula (II) to the compound of formula (I).

Furthermore, it has to be highlighted that the compound of formula (I) is obtained as (*R*) enantiomer with a good enantiomeric excess (abbreviated e.e.).

The process of the present invention is thus based on the reaction of α-angelica lactone of formula (II) with an ene-reductase having SEQ. ID n. 1 or SEQ. ID n. 2 to give (*R*)-β-angelica lactone of formula (I):

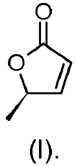

The starting material α-angelica lactone also named 5-methylfuran-2(3H)-one, is commercially available on the market or can be produced according to one the several routes of synthesis disclosed in literature.

The process of the present invention uses enzymes, as catalyst, specifically ene-reductase having SEQ. ID n. 1 or SEQ. ID n. 2 to produce the chiral compound (*R*)-β-angelica lactone of formula (I).

Generally speaking, ene-reductase are enzymes that are metal-independent flavoproteins that catalyse the asymmetric bioreduction of C=C double bonds, using a nicotinamide co-factor as hydride source and the surrounding solvent for proton delivery. In particular, ene-reductases of the Old Yellow Enzyme (OYE) are the flavoprotein known for their reducing activity on activated alkenes, and large number of homologues. These yellow-colored Flavin mononucleotide (FMN) containing enzymes are NAD(P)H dependent. Ene-reductases belonging to the Old Yellow Enzyme (OYE) family exist in variations of their amino acid sequences that render each protein unique in its activity pattern. Some of proteins applied to the reduction of double bonds are reported in the following table.

| Source | Enzyme | Name | Organism | Ref |
|---|---|---|---|---|
| yeasts | OYE1 | Old yellow enzyme 1 | *Saccharomyces pastorianus* | Fig.6 |
| yeasts | OYE2 | Old yellow enzyme 2 | *Saccharomyces cerevisiae* | Fig.7 |
| yeasts | OYE3 | Old yellow enzyme 3 | *Saccharomyces cerevisiae* | Fig.8 |
| bacteria | YqjM | Old yellow enzyme homologue | *Bacillus subtilis* | Fig.9 |
| plants | OPR3 | 12-oxophytod ienoate reductase 3 | *Solanum lycopersicum* | Fig.10 |

In particular, Ene-reductase belonging to the Old Yellow Enzyme (OYEs) family of flavoproteins are known for their broad substrate scope. They are able to reduce a wide range of activated alkenes, such as aldehydes, ketones, nitroalkenes, carboxylic acids and esters, imides and nitriles, employing nicotinamide NAD(P)H as hydride source and are Flavin mononucleotide (FMN), NAD(P)H, dependent catalysts.

According to a preferred embodiment of the process of the present invention, the reaction of α-angelica lactone of formula (II) to give (*R*)-β-angelica lactone of formula (I) occurs with a conversion comprised in the range from 20% to at least 95%.

In particular, when the reaction is carried out with an ene-reductase having SEQ. ID n. 1, conversion is at least 95%. Instead, when the reaction is carried out with an ene-reductase having SEQ. ID n. 2, the conversion is about 20%. Both these reactions are clean since it is not observed the formation of impurities, this means that all starting material, α-angelica lactone is converted only to final product (*R*)-β-angelica lactone.

According to a more preferred embodiment of the process of the present invention, (R)-β-angelica lactone of formula (I) has enantiomeric excess higher than 10% or, at least 70%.

In particular, (*R)*-β-angelica lactone of formula (I) has enantiomeric excess higher than 10%, for example 15%, when the reaction of α-angelica lactone of formula (II) is carried out with the ene-reductase having SEQ. ID n. 2.

According to a preferred embodiment of the process of the present invention, *(R*)-β-angelica lactone of formula (I) has enantiomeric excess of at least 75% and the employed ene-reductase has SEQ. ID n. 1.

The enantiomeric excess or "e.e." or "ee" for short is defined as the absolute difference between the mole fractions of two enantiomers and it is often presented as percent enantiomeric excess, % ee, which is obtained by the following calculation: % ee=|R-S|/|R+S|×100%, wherein the amount of the single enantiomers can be often measured by chiral chromatography.

According to a preferred embodiment of the process of the present invention, the reaction is carried out in presence of tris(hydroxymethyl)aminomethane hydrochloride buffer.

Tris(hydroxymethyl)aminomethane hydrochloride buffer is an aqueous solution containing Tris(hydroxymethyl)aminomethane hydrochloride.

Other buffer solution that could be used to carry out the above mentioned reaction, such as is, for instance, phosphate buffer.

According to a preferred embodiment of the process of the present invention, (*R*)-β-angelica lactone of formula (I) has enantiomeric excess higher than 10% or, at least 70% and the reaction is carried out in presence of tris(hydroxymethyl)aminomethane hydrochloride buffer.

According to a preferred embodiment of the process of the present invention, (*R*)-β-angelica lactone of formula (I) has enantiomeric excess of at least 75% and the ene-reductase having SEQ. ID n. 1, and the reaction is carried out in presence of tris(hydroxymethyl)aminomethane hydrochloride buffer.

According to a preferred embodiment of the process of the present invention, the reaction is carried out at pH value comprised in the range from 5 to 10, preferably in the range from 6 to 9 or more preferably, in the range from 6.5 to 7.5, and again more preferably at pH 7.

The effect provided by different values of pH are evident by the following table:

| | pH 7 | | pH 8 | | pH 9 | |
|---|---|---|---|---|---|---|
| Time(h) | Conv.(%) | ee % | Conv.(%) | ee % | Conv.(%) | ee % |
| 0.5 | - | - | 63 | 77(R) | 90 | 74(R) |
| 2 | 45 | 74(R) | | | | |
| 4 | 65 | 74(R) | | | | |
| 18 | 90 | 74(R) | | | | |

As shown in above table the reaction proceeds faster at higher pH values, i.e. at pH 9 the conversion is already 90 % after only 30 minutes (with ee% of 74(*R*)), instead at pH 7 the conversion is 90 % only after 18 hours.

According to a preferred embodiment of the process of the present invention, (*R*)-β-angelica lactone of formula (I) has enantiomeric excess higher than 10% or, and the reaction is carried out at pH value comprised in the range from 5 to 10, preferably in the range from 6 to 9 or more preferably in the range from 6.5 to 7.5, and again more preferably at pH 7.

According to a more preferred embodiment of the process of the present invention, (*R*)-β-angelica lactone of formula (I) has enantiomeric excess of at least 75% and the ene-reductase having SEQ. ID n. 1, and the reaction is carried out at pH value comprised in the range from 5 to 10, preferably in the range from 6 to 9 or more preferably in the range from 6.5 to 7.5, and again more preferably at pH 7.

The process can be carried out at temperature comprised in the range between 15°C and 50°C.

According to a preferred embodiment of the process of the present invention, the reaction is carried out in a range of temperature comprised between 20°C and 45°C, preferably between 25°C and 35°C or at about 30°C.

According to a preferred embodiment of the process of the present invention, (R)-β-angelica lactone of formula (I) has enantiomeric excess higher than 10%, the reaction is carried out at pH value comprised in the range from 5 to 10, preferably in the range from 6 to 9 or in the range from 6.5 to 7.5, and more preferably at pH 7 and in a range of temperature comprised between 20°C and 45°C.

According to a more preferred embodiment of the process of the present invention, (*R*)-β-angelica lactone of formula (I) has enantiomeric excess higher than 10%, the reaction is carried out at pH value comprised in the range from 5 to 10, preferably in the range from 6 to 9 or in the range from 6.5 to 7.5, and more preferably at pH 7 and in a range of temperature comprised between 25°C and 35°C.

According to a preferred embodiment of the process of the present invention, (*R*)-β-angelica lactone of formula (I) has enantiomeric excess at least 75% and the ene-reductase having SEQ. ID n. 1, the reaction is carried out at pH value comprised in the range from 5 to 10, preferably in the range from 6 to 9 or more preferably in the range from 6.5 to 7.5, and again more preferably at pH 7 and in a range of temperature comprised between 20°C and 45°C.

According to a more preferred embodiment of the process of the present invention, (*R*)-β-angelica lactone of formula (I) has enantiomeric excess at least 75% and the ene-reductase having SEQ. ID n. 1, the reaction is carried out at pH value comprised in the range from 5 to 10, preferably in the range from 6 to 9 or more preferably in the range from 6.5 to 7.5, and again more preferably at pH 7 and in a range of temperature comprised between 25°C and 35°C.

The reaction can be carried out in one phase or in an aqueous biphasic system. In particular, the reaction can be carried out in an aqueous biphasic system.

In particular, aqueous biphasic systems or aqueous two-phase systems is composed of two immiscible liquid phases one being water and the other a water-insoluble organic solvent. The water-insoluble solvent can be selected in a group of hydrocarbon, aromatic hydrocarbon, esters, ketons, ethers, etc..

According to a preferred embodiment of the process of the present invention, the reaction is carried out in an aqueous biphasic system comprising water and ethereal solvent.

Ethereal solvent means that the solvent is an ether compound, i.e. which contains an R-O-R' bond where R and R' and can be the same or different and are C₁-C₅ alkyl group.

In particular, ethereal solvent can be chosen in the group of: Methyl tert-butyl ether (also known as MTBE and tert-butyl methyl ether) Methyltetrahydrofuran (MTHF), Tetrahydrofuran (THF), diethyl ether (CH₃CH₂-O-CH₂CH₃, or Et₂O), dimethoxyethane (DME), etc..

According to a more preferred embodiment of the process of the present invention, the aqueous biphasic system is composed of water and one ethereal solvent chosen in the group of: Methyl tert-butyl ether (MTBE), diethyl ether (Et₂O), tetrahydrofuran (THF) or dimethoxyethane (DME).

According to an again more preferred embodiment of the process of the present invention, the aqueous biphasic system is composed of water and Methyl tert-butyl ether (MTBE).

Preferably, in the above mentioned aqueous biphasic system, the ethereal solvent, in particular Methyl tert-butyl ether (MTBE), can be used in a range comprised between 10% and 50%, specifically at about 10% or 25% or 50%.

In particular, when the process is carried out at pH value higher than 7, for example at pH 8 or 9, it is convenient to carry out the reaction in an aqueous biphasic system, for example that of water and MTBE.

More preferably, when the process is carried out at pH value higher than 7, for example at pH 8 or 9, it is convenient to carry out the reaction in an aqueous biphasic system, wherein said solvent mixture is composed from 10% to 50% of MTBE.

The effect of the presence of the ethereal solvent is to avoid the hydrolysis and/or racemization of (*R*)-β-angelica lactone and to keep the enantiomeric excess stable (as shows in the Example 4).

In particular, Methyl tert-butyl ether solvent (MTBE), provides good conversions of the reaction of α-angelica lactone of formula (II) to β-angelica lactone of formula (I), e.g. conversions comprised in the range from 66% to 84% (see the Example 4).

Another aspect is the process for the preparation of (*R*)-γ-valerolactone of formula (III): 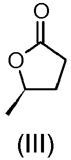 comprising the following steps:
a) preparation of (*R*)-β-angelica lactone of formula (I): 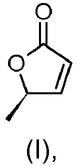 according to the process beforehand described,
b) reduction reaction of the compound of formula (I) obtained in the step a) to give (*R*)-γ-valerolactone of formula (III):

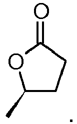

The following scheme shows the above mentioned step a) and step b): 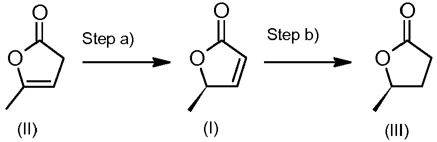 wherein the step a) is carried out with an ene-reductase having SEQ. ID n. 1 or SEQ. ID n. 2.

According to a preferred embodiment of the process of the present invention, the step a) is carried out under the conditions above discussed concerning the process for the preparation of (*R*)-β-angelica lactone of formula (I) from α-angelica lactone of formula (II).

According to a preferred embodiment of the process, (*R*)-γ-valerolactone of formula (III) obtained at the step b) has enantiomeric excess of at least 99%.

According to a preferred embodiment of the process of the present invention, the reduction reaction of the step b) is carried out by two ene-reductases, one being SEQ. ID n. 1 and the other chosen in the group of enzymes: OYE3, YqjM, OPR3 or having SEQ. ID n. 2, or wherein the step b) is carried out by a further addition of ene-reductase having SEQ. ID n. 1.

The following reaction scheme summarizes the enzymatic combinations to carry out the step b):

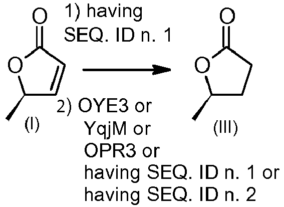

In particular, in the above mentioned group of enzymes, i.e. OYE3, YqjM, OPR3, and others such as OYE2, are known enzyme which are disclosed in some articles, for instance in ChemSusChem (2016), 9, pag. 3393-3396 wherein said enzymes are described and used for bio-enzymatic transformations.

Specifically, OYE3, YqjM, OPR3, OYE2, are all ene-reductases belonging to the Old Yellow Enzyme (OYE) family.

According to a preferred embodiment of the process of the present invention, the reduction reaction of the step b) is carried out by means of Nicotinamide Adenine Dinucleotide Hydrogen (NADH) and/or Nicotinamide Adenine Dinucleotide Phosphate Hydrogen (NAD(P)H).

However, the step b), can alternatively be carried out with chemical reductants, such as, for instance, sodium borohydride, hydrogen in presence of palladium on charcoal, etc..

According to a preferred embodiment of the process of the present invention, the step a) and the step b) are carried out sequentially, without the isolation of the compound of formula (I).

The following reaction scheme shows said sequential process:

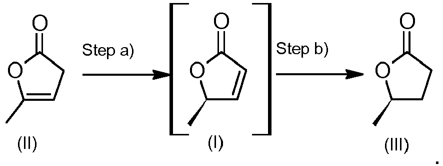

Without isolation means that the step a) and the following step b) are carried out without isolating (*R*)-β-angelica lactone of formula (II) obtained in the step a), therefore starting from R)-β-angelica lactone of formula (II), to get directly (*R*)-γ-valerolactone of formula (III) (see example 7).

According to a preferred embodiment of the process of the present invention, the reduction reaction of the step b) is carried out under the same conditions of buffer solution, pH, temperature, optional biphasic system described above for the conversion of α-angelica lactone of formula (II) to (*R*)-β-angelica lactone of formula (I), i.e. said conditions can be used for the reduction of the compound of formula (I) obtained in the step a) to (*R*)-γ-valerolactone of formula (III).

Another aspect is the process for the preparation of (*R*)-4-hydroxypentanoic acid of formula (IV) or salts thereof: 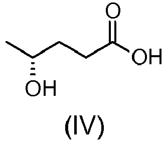 which comprises the following steps:
c) preparation of of (*R*)-γ-valerolactone of formula (III): 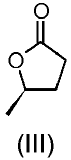 according to the process beforehand described,
d) hydrolysis reaction of the compound of formula (III) obtained in the step c) to give the compound of formula (IV) or salts thereof:

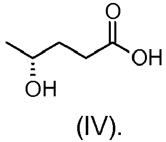

(*R*)-4-Hydroxypentanoic acid of formula (IV) is also known as (*R*)-4-Hydroxyvaleric acid (abbreviated 4R-HVA).

The following scheme summarizes the above mentioned overall process:

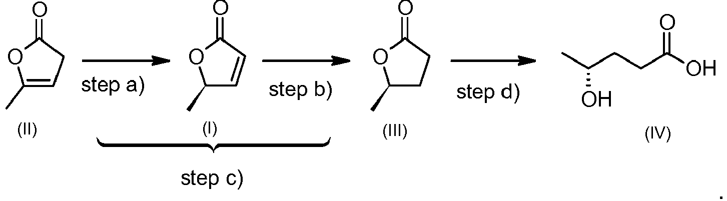

The step c) is carried out according to the processes starting from step a) and proceeding with the step b), under the same conditions of buffer solution, pH, temperature, biphasic system above described for the conversion of α-angelica lactone of formula (II) to (*R*)-β-angelica lactone of formula (I) and from (*R*)-β-angelica lactone of formula (I) to (*R*)-γ-valerolactone of formula (III).

The hydrolysis reaction of the step d) can be carried out in several ways, in particular by means of the following two known ways, both having as starting material (*R*)-γ-valerolactone of formula (III).

The first way to carry out at step d) consists in the ring opening of (*R*)-γ-valerolactone of formula (III) under acidic conditions which leads to the formation of (*R*)-4-hydroxypentanoic acid of formula (IV).

Furthermore, concerning the ring opening/closing, it is known that the compound of formula (IV) forms the compound of formula (III) via ring closure under neutral conditions.

In the other second way to carry out step d) consists in treating (*R*)-γ-valerolactone of formula (III) with NaOH, to give (*R*)-4-hydroxypentanoic acid of formula (IV), as a sodium salt.

According to a preferred embodiment of the process of the present invention, (*R*)-4-hydroxypentanoic acid of formula (IV) has enantiomeric excess of at least 99%.

Further disclosed are the ene-reductases having the sequence SEQ. ID n. 1 or SEQ. ID n. 2.

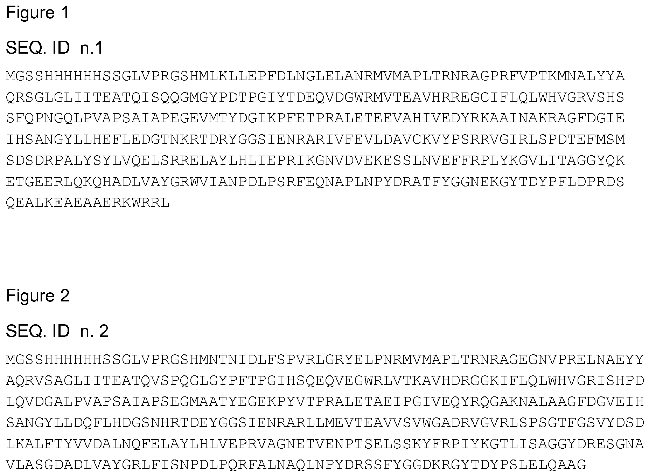

In particular, the ene-reductase having the sequence SEQ. ID n. 1 from Galdieria sulphuraria, has the amino acid sequence as shown in figure 1.

The ene-reductase having the sequence SEQ. ID n. 2 from Chroococcidiopsis thermalis, has the amino acid sequence as shown in figure 2.

Both above mentioned ene-reductases having SEQ. ID n. 1 and SEQ. ID n. 2 are the recombinant enzymes.

Recombinant means that the production organism called host, produces an enzyme based on the DNA sequence encoding said enzyme, belonging to an other donor organism.

Host cell means any cell type that is susceptible to transformation, transfection, transduction or comprising a DNA construct or an expression vector which carries the DNA sequence encoding the enzymes of the present invention, the ene-reductases having SEQ. ID n. 1 and SEQ. ID 2.

The host cell can be a bacterial cell, a fungal cell or an other microorganism cell, specifically, in the present invention the host cell is *Escherichia coli.*

The above mentioned amino acid sequences were expressed starting from the genomic DNA sequences of the above mentioned two organisms and cloned in the vectors pET28A in fusion with the DNA sequence encoding the amino acid tail MGSSHHHHHHSSGLVPRGSH, at the N-terminal end of the fused recombinant protein, comprising a peptide segment consisting of six consecutive histidine (His-tag) so that the sequences therefore results to be SEQ. ID n. 1 and SEQ. ID n. 2.

Both ene-reductases have been expressed in *Escherichia coli* according to the procedures described in the following experimental section. The person skilled in the art will recognize that other cloning vectors might be suitably used, either for bacteria or yeasts, such as, *S. cerevisiae* or *P. pastoris.*

The above mentioned enzymes can be purified by means IMAC chromatography as described in the experimental part.

Moreover, both ene-reductases having SEQ. ID n. 1 and SEQ. ID n. 2 can use NADH and/or NADPH for catalytic activity.

In particular, the ene-reductase having SEQ. ID n. 1 seems to be a monomeric enzyme, and said ene-reductase above mentioned belong to OYE family.

Moreover, the ene-reductase having SEQ. ID n. 1 has the optimum pH of activity comprised in the range between 4 and 9.

Furthermore, said ene-reductase having SEQ. ID n. 1 has a melting point at temperature 67°C, that suggests a good thermo-stability.

Another aspect is the use of the ene-reductases having SEQ. ID n. 1 or SEQ. ID n. 2 for the preparation of (*R*)-β-angelica lactone of formula (I): 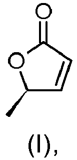 or for the preparation of (*R*)-γ-valerolactone of formula (III):

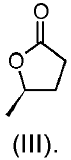

In conclusion, the present invention has realized an unprecedented enantioselective olefin isomerization via biomimetic proton transfer catalysis, by means of new enzymes, i.e. the ene-reductases having SEQ. ID n. 1 or SEQ. ID n. 2.

### EXPERIMENTAL SECTION

The starting material α-angelica lactone is a substance largely commercially available, for example by Sigma-Aldrich (USA).

The following table lists the abbreviations used:
ee = enantiomeric excess
T. = Temperature
h = hour
Eq. = Equivalent
mM = milli Molar
Tris-HCI = tris(hydroxymethyl)aminomethane hydrochloride
MTBE = TBME = tert-butylmethylether
EtOAc = ethyl acetate
DMSO = dimethyl sulfoxide
ON = Over night
OYE = Old Yellow Enzymes
Gs = Galdieria sulphuraria
Ct = Chroococcidiopsis thermalis
GC = Gas Chromatography
GC-MS = Gas Chromatography - Mass Spectrometry
LB = Luria-Bertrani medium
SDS page = Sodium Dodecyl Sulphate - PolyAcrylamide Gel
Electrophoresis
IPTG = isopropyl-β-D-1-tiogalattopiranoside

### Example 1: Synthesis of (R)-β-angelica lactone of formula (I) with the ene-reductase having SEQ. ID n. 1, exemplificative of the invention.

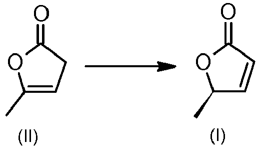

The standard assay (500 µl) was performed in a at 30°C and 120 rpm in 50 mM Tris-HCI (pH 7) containing 10 mM of α-angelica lactone of formula (II). The reaction was started through the addition of ene-reductase enzyme, having SEQ. ID n. 1, to a final concentration of 100 µg/ml and incubated ON.

The substrate was added as a 1 M DMSO solution (1% final DMSO concentration) to overcome its poor solubility in water. After over-night incubation, products were extracted with EtOAc (2 x 0.25 ml) containing 10 mM (*R*)-limonene as internal GC standard. The combined organic phases were dried (Na₂SO₄) and the resulting samples were analysed on GC or GC-MS. Products were identified by comparison with authentic reference materials (which were commercially available) via co-injection on GC or GC-MS.

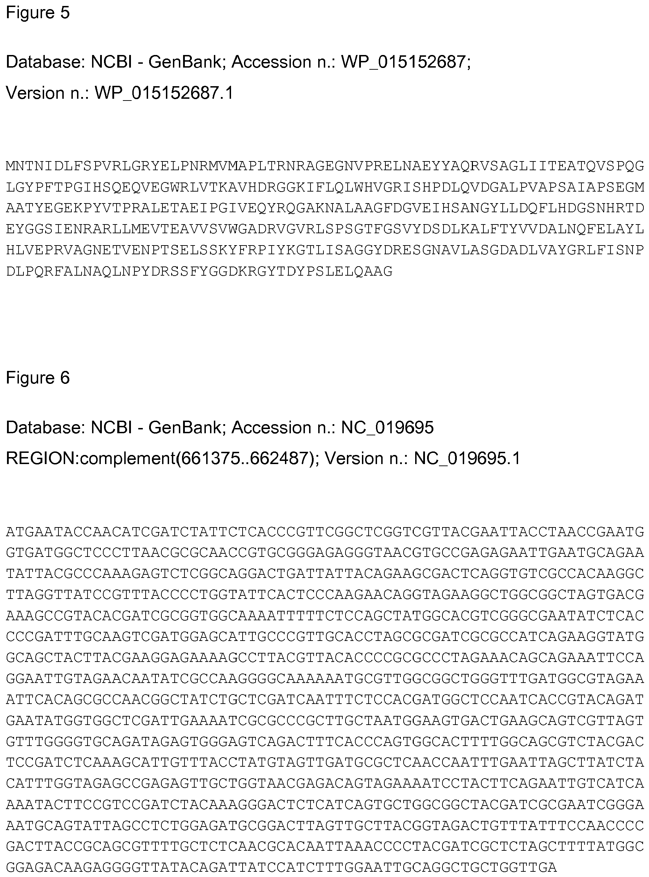

A conversion of 95% to (*R*)-β-angelica lactone of formula (I) (the remaining being of α-angelica lactone) was obtained after 18 hours, and an enantiomeric excess of 75%(*R*). (see the plot of the conversion of Fig. 5.)

### Analytical method to determine the conversion

The mixture was analyzed and the conversion was determined by GC- FID using a 5% phenyl- dimethylpolysiloxane capillary column (HP-5 Agilent, 30 m, 0.32 mm, 0.25 µm), detector temperature 250°C, split ratio 20:1. Programme: 40°C, hold for 2 min, 10°C/min to 180°C, hold for 1 min, 15 °C/min to 280°C hold 1min. Retention times were as follows: α-angelica lactone 5.1 min., β-angelica lactone: 6.29 min. To confirm the identity of the product GC-MS was used with the same programme.

### Analytical method to determine the enantiomeric excess

Enantiomeric excess was determined using a 2,3-di-O-ethyl-6-O-*tert*-butyl dimethylsilyl beta cyclodextrin capillary column (Restek Rt-BDEXse, 30 m x 0.32 mm x 0,25 µm). Detector temperature 200°C, injector temperature 180 °C, split ratio 25:1. Temperature programme: 70°C hold 1 min, 5°C/min to 100°C hold 5 min, 5°C/min to 130°C hold 0 min, 10°C/min to 220°C hold 0 min. Retention times were as follows: α-angelica lactone 7.8 min, (*R*)-β-angelica lactone: 11.51 min, (*S*)-β-angelica lactone: 12.59 min.

### Example 2: Synthesis for the preparation of (R)-β-angelica lactone of formula (I) with the ene-reductase having SEQ. ID n. 1.

The example 1 was repeated, and then conversion and ee were monitored. Table 1 reports the results.

**Table 1**

| | pH7 | |
|---|---|---|
| Time (h) | Conv.(%) | ee (%) |
| 2 | 45 | 74(*R*) |
| 4 | 65 | 74(*R*) |
| 18 | 90 | 74(*R*) |

### Example 3: Synthesis of (R)-β-angelica lactone of formula (I) with an ene-reductase having SEQ. ID n. 2.

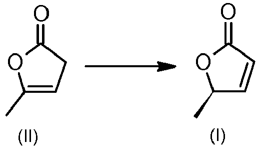

The standard assay (500 µl) was performed in a shaking incubator at 30°C and 120 rpm in 50 mM Tris-HCI (pH 7) containing 10 mM of α-angelica lactone of formula (II). The reaction was started through the addition of ene-reductase enzyme, having SEQ. ID n. 2, to a final concentration of 100 µg /ml and incubated ON.

A conversion of 20% of (*R*)-β-angelica lactone of formula (I) was obtained, and an enantiomeric excess of 15%(*R*).

The substrate was added as a 1 M DMSO solution (1% final DMSO concentration) to overcome its poor solubility in water. After over-night incubation, products were extracted with EtOAc (2 x 0.25 ml) containing 10 mM (R)-limonene as internal GC standard. The combined organic phases were dried (Na₂SO₄) and the resulting samples were analysed on GC or GC-MS. Products were identified by comparison with authentic reference materials (which were commercially available) via co-injection on GC or GC-MS.

The conversion and the enantiomeric excess were determined by the analytical methods described in example 1.

### Example 4: Synthesis of (R)-β-angelica lactone of formula (I) with the ene-reductase having SEQ. ID n. 1 and in bi-phasic system.

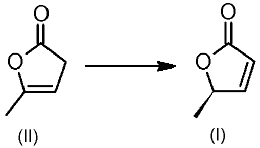

The standard assay (500 µl) was performed at 30°C and 120 rpm in 50 mM Tris-HCI (pH 7 or pH 9) containing 10 mM of α-angelica lactone of formula (II) and in presence of the following amount of volume of TBME. The reaction was started through the addition of ene-reductase enzyme, having SEQ. ID n. 1, to a final concentration of 100 µg/ml and incubate ON.

The following Table 2 reports the results in terms of conversion and ee achieved using various percentages of TBME and carried out at pH 7.

**Table 2**

| %TBME | Conv. (*R*)-β-angelica lactone | ee% |
|---|---|---|
| 10 | 84 | 74 (*R*) |
| 25 | 76 | 73 (*R*) |
| 50 | 66 | 73 (*R*) |

### Example 5: Synthesis of (R)-γ-valerolactone of formula (III) with an ene-reductase having SEQ. ID n. 1.

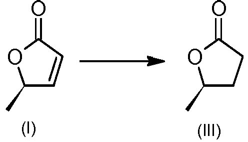

Starting from the (*R*)-β-angelica lactone of formula (I) as obtained in the example 1, 15 mM of NADH and 100 µg /ml of ene-reductase enzyme, having SEQ. ID n. 1, were added and left incubate for 3h at 30°C and 120 rpm.

A conversion of 27% of (*R*)-γ-valerolactone of formula (III) was obtained after 3 hours at 30°C, with an enantiomeric excess of 99%(*R*).

After incubation, products were extracted with EtOAc (2 x 0.25 ml) containing 10 mM (R)-limonene as internal GC standard. The combined organic phases were dried (Na₂SO₄) and the resulting samples were analysed on GC or GC-MS. Products were identified by comparison with authentic reference materials (which were commercially available) via co-injection on GC or GC-MS.

The conversion and the enantiomeric excesses were determined using a 2,3-di-O-ethyl-6-O-*tert*-butyl dimethylsilyl beta cyclodextrin capillary column (Restek Rt-BDEXse, 30 m x 0.32 mm x 0,25 µm). Detector temperature 200°C, injector temperature 180 °C, split ratio 25:1. Temperature programme: 70°C hold 1min, 5°C/min to 100°C hold 5 min, 5°C/min to 130°C hold 0, 10°C/min to 220°C hold 0 min. Retention times were as follows: α-angelica lactone 7.8 min, (*R*)-β-angelica lactone: 11.51 min, (*S*)-β-angelica lactone: 12.59 min, (*R*)-γ-valerolactone: 11.4 min, (*S*)-γ-valerolactone: 12.77 min.

### Example 6: Synthesis of (R)-γ-valerolactone of formula (III), without the isolation of the compound of formula (I), with further the addition of ene-reductase, having SEQ. ID n. 1

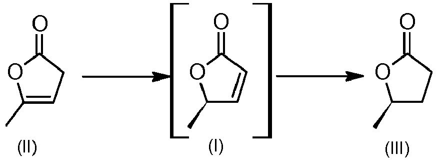

The standard assay (500 µl) was performed in a shaking incubator at 30°C and 120 rpm in 50 mM Tris-HCI (pH 7) containing 10 mM α-angelica lactone of formula (II). The reaction of isomerization was started through the addition of ene-reductase enzyme, having SEQ. ID n. 1 to a final concentration of 100 µg/ml and incubate ON. After the ON incubation, and in particular, without the isolation of the compound of formula (I), were added 15 mM of NADH and 100 µg/ml of ene-reductase enzyme, having SEQ. ID n. 1, and let incubate for 3h at 30°C and 120 rpm in order to achieve the reduction of the C=C bond.

A conversion of 27% of (*R*)-γ-valerolactone of formula (III) was obtained, with an enantiomeric excess of 99%(*R*).

### Example 7: Synthesis of (R)-γ-valerolactone of formula (III), without the isolation of the compound of formula (I), with two ene-reductases, wherein one having SEQ. ID n. 1 and the other was chosen in the group of enzymes: OYE1, OYE2, OYE3, YqjM, OPR3 or having SEQ. ID n. 2.

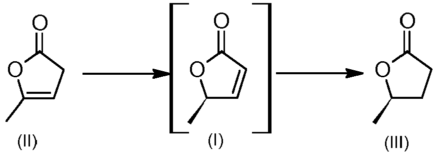

The standard assay (500 µl) was performed in a shaking incubator at 30°C and 120 rpm in 50 mM Tris-HCl (pH 7) containing 10 mM of (α-angelica lactone of formula (II). The reaction was started through the addition of ene-reductase enzyme, having SEQ. ID n. 1 to a final concentration of and incubate ON. After the ON incubation, and in particular, without the isolation of the compound of formula (I), were added 15 mM of NADH and 100 µg/ml of ene-reductase enzyme, chosen in the group of enzymes: OYE1, OYE2, OYE3, YqjM, OPR3 or having SEQ. ID n. 2, and let incubate for 3h at 30°C and 120 rpm.

The following Table 3 reports the results with the use of several ene-reductase enzymes.

**Table 3**

| Enzymes | ee (*R*)-β-angelica lactone of formula (I) (%) | ee (*R*)-γ-valerolactone of formula (III) (%) |
|---|---|---|
| SEQ. ID n. 1 + SEQ. ID n. 2 | 73 (R) | >99 (R) |
| SEQ. ID n. 1 + OYE1 | 22 (R) | 74 (R) |
| SEQ. ID n. 1 + OYE2 | 14 (R) | 75 (R) |
| SEQ. ID n. 1 + OYE3 | 70 (R) | >99 (R) |
| SEQ. ID n. 1 + OPR3 | 61 (R) | >99 (R) |
| SEQ. ID n. 1 + YqjM | 40 (R) | >99 (R) |

### Example 8: Synthesis of sodium salt of (R)-4-hydroxypentanoic acid of formula (IV).

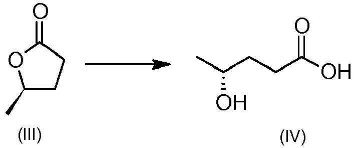

The (*R*)-γ-valerolactone of formula (III) (1.00 g, 10 mmol) was heated at 50°C for 24 h with 2 N NaOH (5mL). The mixture was cooled and concentrated *in vacuo* to give a wet gray solid. The product is the sodium salt of (*R*)-4-hydroxypentanoic acid of formula (IV) was determined to be >95:5 R/S.

### Example 9: Preparation of ene-reductase enzymes having SEQ. ID n. 1 and SEQ. ID n. 2. (falling outside the scope of the invention)

The recombinant enzymes *Gs*OYE having SEQ. ID n. 1 and CtOYE having SEQ. ID n. 2 were expressed in E. coli BL21 (DE3). Pre-cultures were carried out in 20 ml Luria-Bertani (LB) medium at 37°C containing 50 µg/ml kanamycin. Larger cultures were carried out in 1 L LB medium with riboflavin addition (25 µM final concentration). The cells were grown in a shaking incubator at 37°C to an optical density at 600 nm (OD₆₀₀) of 0.4-0.6, then induced by addition of isopropyl-β-D-1-tiogalattopiranoside

(IPTG) to a final concentration of 0.2 mM and cultivated at 25°C overnight. The cells were harvested by centrifugation (4°C, 10 min, 4500 × g) and washed with 50 mM Tris-HCI buffer pH 8.0. Cell disruption was obtained by French Press and crude extract was centrifuged (4°C, 30 min, 18,000 × g) to separate soluble and insoluble fractions. To enhance the flavination FMN cofactor (at 100 µM final concentration) was added to the crude extract before cell disruption. Expression of recombinant protein was checked by Sodium Dodecyl Sulphate - PolyAcrylamide Gel Electrophoresis (SDS-PAGE) analysis.

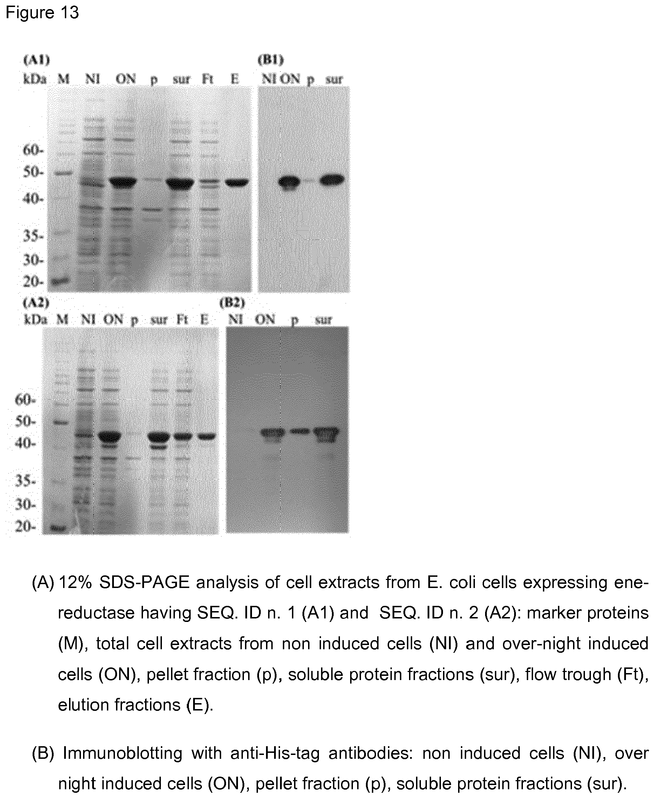

The identity of the proteins was verified by immunoblotting using anti His-tag antibodies (Figure 13). Overexpressed proteins were purified by immobilized-metal affinity chromatography (IMAC). Soluble fractions obtained from 1 L culture were incubated with the resin for 30 min at 4°C and then loaded on a 5 ml column (Bio-Rad). The column was washed by gravitational flow with five column volumes of 50 mM Tris-HCI buffer pH 8.0. Elution was performed by five column volumes of 50 mM Tris-HCI pH 8.0, 250 mM imidazole solution. Enzyme concentration was measured by spectrophotometry, measuring concentration of free flavin in a solution of denatured protein. Yields of 98 mg/L and 80 mg/L were obtained for GsOYE and CtOYE, respectively.

### Organisms and culture conditions for enzymes DNA sequence isolation

Galdieria sulphuraria strain 074W was kindly provided by Dr. Antonino Pollio from the ACUF collection of the Biological Science Department of University Federico II, Naples, Italy. Cultures of G. sulphuraria were grown at 30°C in Allen medium pH 1, in 500 ml flasks on a rotatory platform shaker at 70 rpm. Light conditions used were 25 mol photons m-2 sec-1 .

*Chroccocidiopsis thermalis* PCC 7203 was obtained from the Pasteur Culture Collection (Paris, France). Cells were grown photoautotrophically at 20°C degrees in BG11 medium. Static cultures were continuously illuminated at 25 mol photons m⁻² sec⁻¹.

The NCBI resource was used for DNA sequence analysis; searches and multiple alignments of *Gs*OYE and *Ct*OYE sequences were respectively produced by programs BLAST and Clustal Omega.

Genomic DNA of the cultured organisms was obtained following the DNA extraction method reported by Allen et al. (Allen, G. C., et al. "A modified protocol for rapid DNA isolation from plant tissues using cetyltrimethylammonium bromide." Nature protocols 1.5 (2006): 2320.)

Expression vectors were produced by digestion of pET-28a(+) with NdeI/BamHI and ligation of the amplified *Gs*OYE and *Ct*OYE sequences, cut by the same enzymes. These sequences were obtained by two subsequent PCR reactions, the first one producing a preliminary "large" amplimer, which was then used for the second amplification, by nested mutagenic primers. The following synthetic oligonucleotides were used for PCR amplification of the target gene from *G. sulphuraria* genomic DNA: external primer pair, forward 5'-CGTCCGTTGTAGTTAGTGGACGGT-3' and reverse 5'-TGCGAGTCATCCAACAGAACAACT-3'; nested primer pair, forward 5'-TGGACGGTGACATATGTTGAAGC-3' and reverse 5'-ATAGTTTTGGATCCTTTGTGGAAGAC-3'.

The following synthetic oligonucleotides were used for PCR amplification of the target gene from *C. thermalis* genomic DNA: external primer pair, forward 5'-CAATTTTCAATCTGGTGGGGTCGGC-3' and reverse 5' - ACAGTTGCGATCGAGTAGGATTCGC 3'; nested primer pair, forward 5'-CATTTACCCTAGTAAAGCATATGAATACCAACATCG-3' and reverse 5'-ATTAGGATCCTCAACCAGCAGCCTGCAATTCCAAAG-3'.

## Claims

1. Process for the preparation of (*R*)-β-angelica lactone of formula (I): 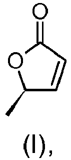 by means of the reaction of α-angelica lactone of formula (II): 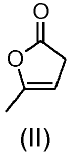 with an ene-reductase having SEQ. ID n. 1 or SEQ. ID n. 2.

2. Process according to the claim 1, wherein the (*R*)-β-angelica lactone of formula (I) has enantiomeric excess higher than 10% or, at least 70%.

3. Process according to the claim 1, wherein the (*R*)-β-angelica lactone of formula (I) has enantiomeric excess of at least 75% and the ene-reductase has SEQ. ID n. 1.

4. Process according to any one of the claims from 1 to 3, wherein the reaction is carried out in presence of tris(hydroxymethyl)aminomethane hydrochloride buffer.

5. Process according to any one of the claims from 1 to 4, wherein the reaction is carried out at pH value comprised in the range from 6 to 9 or in the range from 6.5 to 7.5.

6. Process according to any one of the claims from 1 to 5, wherein the reaction is carried out in a range of temperature comprised between 25°C and 35°C or at about 30°C.

7. Process according to any one of the claims from 1 to 6, wherein the reaction is carried out in an aqueous biphasic system comprising water and an ethereal solvent.

8. Process for the preparation of (*R*)-γ-valerolactone of formula (III): 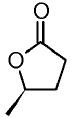 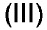 comprising the following steps:
a) preparation of (*R*)-β-angelica lactone of formula (I): 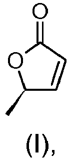 according to the process of any one of the claims from 1 to 7,
b) reduction reaction of the compound of formula (I) obtained in the step a) to give (*R*)-γ-valerolactone of formula (III):

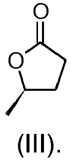

9. Process according to the claim 8, wherein the (*R*)-γ-valerolactone of formula (III) has enantiomeric excess of at least 99%.

10. Process according to any one of the claims from 8 to 9, wherein the reduction reaction of the step b) is carried out by two ene-reductases, one being SEQ. ID n. 1 and the other chosen in the group of enzymes: OYE3, YqjM, OPR3 or having SEQ. ID n. 2, or wherein the step b) is carried out by a further addition of ene-reductase having SEQ. ID n. 1.

11. Process according to any one of the claims from 8 to 10, wherein the reduction reaction of the step b) is carried out by means of NADH and/or NAD(P)H.

12. Process according to any one of the claims from 8 to 11, wherein the step a) and the step b) are carried out sequentially, without the isolation of the compound of formula (I).

13. Process according to any one of the claims from 8 to 12, wherein the reduction reaction of the step b) is carried out under the same conditions claimed in any one of the claims from 3 to 7.

14. Process for the preparation of (*R*)-4-hydroxypentanoic acid of formula (IV) or salts thereof: 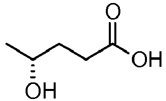 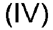 comprising the following steps:
c) preparation of (*R*)-γ-valerolactone of formula (III): 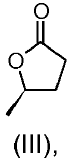 according to the process of any one of the claims from 8 to 13,
d) hydrolysis reaction of the compound of formula (III) obtained in the step c) to give the compound of formula (IV) or salts thereof:

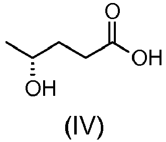

15. Process according to the claims 14, wherein the compound of formula (IV) has enantiomeric excess of at least 99%.

16. Use of the ene-reductases having SEQ. ID n. 1 or SEQ. ID n. 2 for the preparation of *(R*)-β-angelica lactone of formula (I): 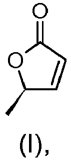 or for the preparation of (*R*)-γ-valerolactone of formula (III):

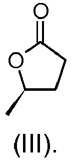

## Patentansprüche

1. Verfahren zur Herstellung von (*R*)-β-Angelicalacton mit der Formel (I): 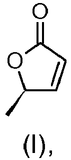 mittels der Reaktion von α-Angelicalacton mit der Formel (II): 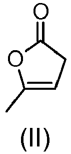 mit einer En-Reduktase mit SEQ. ID. Nr. 1 oder SEQ. ID. Nr. 2.

2. Verfahren nach Anspruch 1, wobei das (*R*)-β-Angelicalacton mit der Formel (I) einen Enantiomerenüberschuss von mehr als 10 % oder mindestens 70 % aufweist.

3. Verfahren nach Anspruch 1, wobei das (*R*)-β-Angelicalacton mit der Formel (I) einen Enantiomerenüberschuss mindestens 75 % aufweist und die En-Reduktase SEQ. ID. Nr. 1 hat.

4. Verfahren nach einem der Ansprüche 1 bis 3, wobei die Reaktion in Gegenwart von Tris(hydroxymethyl)aminomethan-Hydrochlorid-Puffer durchgeführt wird.

5. Verfahren nach einem der Ansprüche 1 bis 4, wobei die Reaktion bei einem pH-Wert durchgeführt wird, der im Bereich von 6 bis 9 oder im Bereich von 6,5 bis 7,5 liegt.

6. Verfahren nach einem der Ansprüche 1 bis 5, wobei die Reaktion in einem Temperaturbereich durchgeführt wird, der zwischen 25 °C und 35 °C liegt, oder bei etwa 30 °C.

7. Verfahren nach einem der Ansprüche 1 bis 6, wobei die Reaktion in einem wässrigen Zweiphasensystem durchgeführt wird, das Wasser und ein etherisches Lösungsmittel umfasst.

8. Verfahren zur Herstellung von (*R*)-γ-Valerolacton mit der Formel (III): 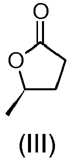 umfassend die folgenden Schritte:
a) Herstellung von (*R*)-β-Angelicalacton mit der Formel (I): 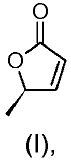 gemäß dem Verfahren nach einem der Ansprüche 1 bis 7,
b) Reduktionsreaktion der in Schritt a) erhaltenen Verbindung mit der Formel (I), um (*R*)-γ-Valerolacton mit der Formel (III) zu ergeben:

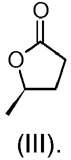

9. Verfahren nach Anspruch 8, wobei das (*R*)-γ-Valerolacton mit der Formel (III) einen Enantiomerenüberschuss von mindestens 99 % aufweist.

10. Verfahren nach einem der Ansprüche 8 bis 9, wobei die Reduktionsreaktion von Schritt b) durch zwei En-Reduktasen durchgeführt wird, wobei eine SEQ. ID. Nr. 1 ist, und die andere ausgewählt ist aus der Gruppe von Enzymen: OYE3, YqjM, OPR3, oder SEQ. ID. Nr. 2 aufweist, oder wobei der Schritt b) durch eine weitere Zugabe von En-Reduktase mit SEQ. ID. Nr. 1 durchgeführt wird.

11. Verfahren nach einem der Ansprüche 8 bis 10, wobei die Reduktionsreaktion von Schritt b) mittels NADH und/oder NAD(P)H durchgeführt wird.

12. Verfahren nach einem der Ansprüche 8 bis 11, wobei der Schritt a) und der Schritt b) sequentiell ohne Isolierung der Verbindung mit der Formel (I) durchgeführt werden.

13. Verfahren nach einem der Ansprüche 8 bis 12, wobei die Reduktionsreaktion von Schritt b) unter denselben Bedingungen durchgeführt wird, die in einem der Ansprüche 3 bis 7 beansprucht werden.

14. Verfahren zur Herstellung von (*R*)-4-Hydroxypentansäure mit der Formel (IV) oder deren Salzen: 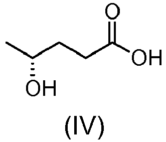 umfassend die folgenden Schritte:
c) Herstellung von (*R*)-γ-Valerolacton mit der Formel (III): 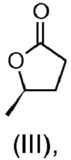 gemäß dem Verfahren nach einem der Ansprüche 8 bis 13,
d) Hydrolysereaktion der in Schritt c) erhaltenen Verbindung mit der Formel (III), um die Verbindung mit der Formel (IV) oder Salze davon zu ergeben:

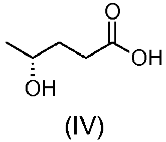

15. Verfahren nach Anspruch 14, wobei die Verbindung mit der Formel (IV) einen Enantiomerenüberschuss von mindestens 99 % aufweist.

16. Verwendung der En-Reduktasen mit SEQ. ID. Nr. 1 oder SEQ. ID. Nr. 2 zur Herstellung von *(R*)-β-Angelicalacton mit der Formel (I): 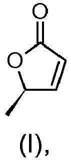 oder zur Herstellung von (*R*)-γ-Valerolacton mit der Formel (III):

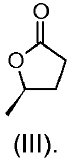

## Revendications

1. Procédé de préparation de (*R*)-β-angelica lactone de formule (I) : 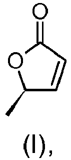 au moyen de la réaction de l'a-angelica lactone de formule (II) : 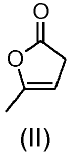 avec une ène réductase présentant la séquence SEQ. ID. NO 1 ou la séquence SEQ. ID. NO 2.

2. Procédé selon la revendication 1, la (*R*)-β-angelica lactone de formule (I) présentant un excès énantiomérique supérieur à 10% ou d'au moins 70%.

3. Procédé selon la revendication 1, la (*R*)-β-angelica lactone de formule (I) présentant un excès énantiomérique d'au moins 75% et l'ène réductase présentant la séquence SEQ. ID. NO 1.

4. Procédé selon l'une quelconque des revendications 1 à 3, la réaction étant réalisée en présence d'un tampon de chlorhydrate de tris(hydroxyméthyl)aminométhane.

5. Procédé selon l'une quelconque des revendications 1 à 4, la réaction étant réalisée à une valeur de pH comprise dans la plage de 6 à 9 ou dans la plage de 6,5 à 7,5.

6. Procédé selon l'une quelconque des revendications 1 à 5, la réaction étant réalisée dans une plage de température comprise entre 25°C et 35°C ou à environ 30°C.

7. Procédé selon l'une quelconque des revendications 1 à 6, la réaction étant réalisée dans un système biphasique aqueux comprenant de l'eau et un solvant éthéré.

8. Procédé de préparation de (*R*)-γ-valérolactone de formule (III) : 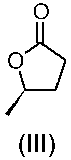 comprenant les étapes suivantes :
a) préparation de (*R*)-β-angelica lactone de formule (I) : 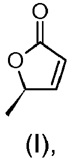 selon le procédé de l'une quelconque des revendications 1 à 7,
b) réaction de réduction du composé de formule (I) obtenu à l'étape a) pour donner la (*R*)-γ-valérolactone de formule (III) :

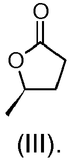

9. Procédé selon la revendication 8, la (*R*)-γ-valérolactone de formule (III) présentant un excès énantiomérique d'au moins 99%.

10. Procédé selon l'une quelconque des revendications 8 à 9, la réaction de réduction de l'étape b) étant réalisée par deux ène réductases, l'une étant de séquence SEQ. ID. NO 1 et l'autre étant choisie dans le groupe des enzymes : OYE3, YqjM, OPR3 ou présentant la séquence SEQ. ID. NO 2 ou l'étape b) étant réalisée par une addition supplémentaire d'ène réductase présentant la séquence SEQ. ID. NO 1.

11. Procédé selon l'une quelconque des revendications 8 à 10, la réaction de réduction de l'étape b) étant réalisée au moyen de NADH et/ou de NAD(P)H.

12. Procédé selon l'une quelconque des revendications 8 à 11, l'étape a) et l'étape b) étant réalisées de manière séquentielle, sans l'isolement du composé de formule (I).

13. Procédé selon l'une quelconque des revendications 8 à 12, la réaction de réduction de l'étape b) étant réalisée dans les mêmes conditions que celles revendiquées dans l'une quelconque des revendications 3 à 7.

14. Procédé de préparation d'acide (*R*)-4-hydroxypentanoïque de formule (IV) ou de sels de celui-ci : 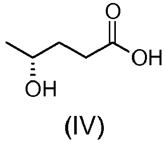 comprenant les étapes suivantes :
c) préparation de (*R*)-γ-valérolactone de formule (III) : 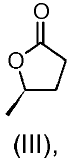 selon le procédé de l'une quelconque des revendications 8 à 13,
d) réaction d'hydrolyse du composé de formule (III) obtenu à l'étape c) pour donner le composé de formule (IV) ou de sels de celui-ci :

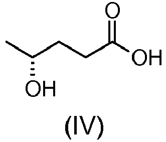

15. Procédé selon la revendication 14, le composé de formule (IV) présentant un excès énantiomérique d'au moins 99%.

16. Utilisation des ène réductases présentant la séquence SEQ. ID. NO 1 ou la séquence SEQ. ID. NO 2 pour la préparation de *(R*)-β-angelica lactone de formule (I) : 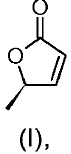 ou pour la préparation de (*R*)-γ-valérolactone de formule (III) :


### SEQUENCE LISTING

<110> F.I.S. - Fabbrica Italiana Sintetici S.p.A.
<120> Efficient process for the preparation of (R)-Î²-angelica lactone and
   derivatives thereof
<130> 0063/EP/PRI
<140> EP17189155.9
   <141> 2017-09-04
<160> 11
<170> BiSSAP 1.3.6
<210> 1
   <211> 401
   <212> PRT
   <213> Galdieria sulphuraria
<400> 1
   Met Gly Ser Ser His His His His His His Ser Ser Gly Leu Val Pro
   1               5                   10                  15
   Arg Gly Ser His Met Leu Lys Leu Leu Glu Pro Phe Asp Leu Asn Gly
               20                  25                  30
   Leu Glu Leu Ala Asn Arg Met Val Met Ala Pro Leu Thr Arg Asn Arg
           35                  40                  45
   Ala Gly Pro Arg Phe Val Pro Thr Lys Met Asn Ala Leu Tyr Tyr Ala
       50                  55                  60
   Gln Arg Ser Gly Leu Gly Leu Ile Ile Thr Glu Ala Thr Gln Ile Ser
   65                  70                  75                  80
   Gln Gln Gly Met Gly Tyr Pro Asp Thr Pro Gly Ile Tyr Thr Asp Glu
                   85                  90                  95
   Gln Val Asp Gly Trp Arg Met Val Thr Glu Ala Val His Arg Arg Glu
               100                 105                 110
   Gly Cys Ile Phe Leu Gln Leu Trp His Val Gly Arg Val Ser His Ser
           115                 120                 125
   Ser Phe Gln Pro Asn Gly Gln Leu Pro Val Ala Pro Ser Ala Ile Ala
       130                 135                 140
   Pro Glu Gly Glu Val Met Thr Tyr Asp Gly Ile Lys Pro Phe Glu Thr
   145                 150                 155                 160
   Pro Arg Ala Leu Glu Thr Glu Glu Val Ala His Ile Val Glu Asp Tyr
                   165                 170                 175
   Arg Lys Ala Ala Ile Asn Ala Lys Arg Ala Gly Phe Asp Gly Ile Glu
               180                 185                 190
   Ile His Ser Ala Asn Gly Tyr Leu Leu His Glu Phe Leu Glu Asp Gly
           195                 200                 205
   Thr Asn Lys Arg Thr Asp Arg Tyr Gly Gly Ser Ile Glu Asn Arg Ala
       210                 215                 220
   Arg Ile Val Phe Glu Val Leu Asp Ala Val Cys Lys Val Tyr Pro Ser
   225                 230                 235                 240
   Arg Arg Val Gly Ile Arg Leu Ser Pro Asp Thr Glu Phe Met Ser Met
                   245                 250                 255
   Ser Asp Ser Asp Arg Pro Ala Leu Tyr Ser Tyr Leu Val Gln Glu Leu
               260                 265                 270
   Ser Arg Arg Glu Leu Ala Tyr Leu His Leu Ile Glu Pro Arg Ile Lys
           275                 280                 285
   Gly Asn Val Asp Val Glu Lys Glu Ser Ser Leu Asn Val Glu Phe Phe
       290                 295                 300
   Arg Pro Leu Tyr Lys Gly Val Leu Ile Thr Ala Gly Gly Tyr Gln Lys
   305                 310                 315                 320
   Glu Thr Gly Glu Glu Arg Leu Gln Lys Gln His Ala Asp Leu Val Ala
                   325                 330                 335
   Tyr Gly Arg Trp Val Ile Ala Asn Pro Asp Leu Pro Ser Arg Phe Glu
               340                 345                 350
   Gln Asn Ala Pro Leu Asn Pro Tyr Asp Arg Ala Thr Phe Tyr Gly Gly
           355                 360                 365
   Asn Glu Lys Gly Tyr Thr Asp Tyr Pro Phe Leu Asp Pro Arg Asp Ser
       370                 375                 380
   Gln Glu Ala Leu Lys Glu Ala Glu Ala Ala Glu Arg Lys Trp Arg Arg
   385                 390                 395                 400
   Leu
<210> 2
   <211> 390
   <212> PRT
   <213> Chroococcidiopsis thermalis
<400> 2
   Met Gly Ser Ser His His His His His His Ser Ser Gly Leu Val Pro
   1               5                   10                  15
   Arg Gly Ser His Met Asn Thr Asn Ile Asp Leu Phe Ser Pro Val Arg
               20                  25                  30
   Leu Gly Arg Tyr Glu Leu Pro Asn Arg Met Val Met Ala Pro Leu Thr
           35                  40                  45
   Arg Asn Arg Ala Gly Glu Gly Asn Val Pro Arg Glu Leu Asn Ala Glu
       50                  55                  60
   Tyr Tyr Ala Gln Arg Val Ser Ala Gly Leu Ile Ile Thr Glu Ala Thr
   65                  70                  75                  80
   Gln Val Ser Pro Gln Gly Leu Gly Tyr Pro Phe Thr Pro Gly Ile His
                   85                  90                  95
   Ser Gln Glu Gln Val Glu Gly Trp Arg Leu Val Thr Lys Ala Val His
               100                 105                 110
   Asp Arg Gly Gly Lys Ile Phe Leu Gln Leu Trp His Val Gly Arg Ile
           115                 120                 125
   Ser His Pro Asp Leu Gln Val Asp Gly Ala Leu Pro Val Ala Pro Ser
       130                 135                 140
   Ala Ile Ala Pro Ser Glu Gly Met Ala Ala Thr Tyr Glu Gly Glu Lys
   145                 150                 155                 160
   Pro Tyr Val Thr Pro Arg Ala Leu Glu Thr Ala Glu Ile Pro Gly Ile
                   165                 170                 175
   Val Glu Gln Tyr Arg Gln Gly Ala Lys Asn Ala Leu Ala Ala Gly Phe
               180                 185                 190
   Asp Gly Val Glu Ile His Ser Ala Asn Gly Tyr Leu Leu Asp Gln Phe
           195                 200                 205
   Leu His Asp Gly Ser Asn His Arg Thr Asp Glu Tyr Gly Gly Ser Ile
       210                 215                 220
   Glu Asn Arg Ala Arg Leu Leu Met Glu Val Thr Glu Ala Val Val Ser
   225                 230                 235                 240
   Val Trp Gly Ala Asp Arg Val Gly Val Arg Leu Ser Pro Ser Gly Thr
                   245                 250                 255
   Phe Gly Ser Val Tyr Asp Ser Asp Leu Lys Ala Leu Phe Thr Tyr Val
               260                 265                 270
   Val Asp Ala Leu Asn Gln Phe Glu Leu Ala Tyr Leu His Leu Val Glu
           275                 280                 285
   Pro Arg Val Ala Gly Asn Glu Thr Val Glu Asn Pro Thr Ser Glu Leu
       290                 295                 300
   Ser Ser Lys Tyr Phe Arg Pro Ile Tyr Lys Gly Thr Leu Ile Ser Ala
   305                 310                 315                 320
   Gly Gly Tyr Asp Arg Glu Ser Gly Asn Ala Val Leu Ala Ser Gly Asp
                   325                 330                 335
   Ala Asp Leu Val Ala Tyr Gly Arg Leu Phe Ile Ser Asn Pro Asp Leu
               340                 345                 350
   Pro Gln Arg Phe Ala Leu Asn Ala Gln Leu Asn Pro Tyr Asp Arg Ser
           355                 360                 365
   Ser Phe Tyr Gly Gly Asp Lys Arg Gly Tyr Thr Asp Tyr Pro Ser Leu
       370                 375                 380
   Glu Leu Gln Ala Ala Gly
   385                 390
<210> 3
   <211> 20
   <212> PRT
   <213> Artificial Sequence
<220>
   <223> Peptide segment
<400> 3
   Met Gly Ser Ser His His His His His His Ser Ser Gly Leu Val Pro
   1               5                   10                  15
   Arg Gly Ser His
               20
<210> 4
   <211> 24
   <212> DNA
   <213> Artificial Sequence
<220>
   <223> External primer forward
<400> 4
   cgtccgttgt agttagtgga cggt 24
<210> 5
   <211> 24
   <212> DNA
   <213> Artificial Sequence
<220>
   <223> External primer reverse
<400> 5
   tgcgagtcat ccaacagaac aact 24
<210> 6
   <211> 23
   <212> DNA
   <213> Artificial Sequence
<220>
   <223> Nested primer forward
<400> 6
   tggacggtga catatgttga agc 23
<210> 7
   <211> 26
   <212> DNA
   <213> Artificial Sequence
<220>
   <223> Nested primer reverse
<400> 7
   atagttttgg atcctttgtg gaagac 26
<210> 8
   <211> 25
   <212> DNA
   <213> Artificial Sequence
<220>
   <223> External primer forward
<400> 8
   caattttcaa tctggtgggg tcggc 25
<210> 9
   <211> 25
   <212> DNA
   <213> Artificial Sequence
<220>
   <223> External primer reverse
<400> 9
   acagttgcga tcgagtagga ttcgc 25
<210> 10
   <211> 36
   <212> DNA
   <213> Artificial Sequence
<220>
   <223> Nested primer forward
<400> 10
   catttaccct agtaaagcat atgaatacca acatcg 36
<210> 11
   <211> 36
   <212> DNA
   <213> Artificial Sequence
<220>
   <223> Nested primer reverse
<400> 11
   attaggatcc tcaaccagca gcctgcaatt ccaaag 36